Europäisches Patentamt

European Patent Office

Office européen des brevets

(11) Publication number: **0 161 901 B1**

(12) **EUROPEAN PATENT SPECIFICATION**

(45) Date of publication of patent specification: **01.12.93**

(21) Application number: **85303234.0**

(22) Date of filing: **07.05.85**

Divisional application 93104177.6 filed on 07/05/85.

(51) Int. Cl.⁵: **C12N 15/25**, C12P 21/02, C07K 13/00, C12N 1/20, C12N 5/00, //(C12N1/20, C12R1:19),(C12N5/00, C12R1:91)

(54) Human il-1 cDNA sequences encoding biologically-active human il-1 proteins.

(30) Priority: **18.05.84 US 611669**
**11.02.85 US 700374**

(43) Date of publication of application:
**21.11.85 Bulletin 85/47**

(45) Publication of the grant of the patent:
**01.12.93 Bulletin 93/48**

(84) Designated Contracting States:
**BE CH DE FR GB IT LI NL SE**

(56) References cited:
**EP-A- 0 165 654**
**EP-A- 0 188 864**
**EP-A- 0 188 920**
**FR-A- 2 519 553**

(73) Proprietor: **NEW ENGLAND MEDICAL CENTER HOSPITALS, INC.**
**171 Harrison Avenue**
**Boston, MA 02111(US)**

Proprietor: **THE TRUSTEES OF TUFTS COLLEGE**
**136 Harrison Avenue**
**Boston Massachusetts(US)**

Proprietor: **WELLESLEY COLLEGE**

**Wellesley Massachusetts(US)**

Proprietor: **MASSACHUSETTS INSTITUTE OF TECHNOLOGY**
**77 Massachusetts Avenue**
**Cambridge, MA 02139(US)**

(72) Inventor: **Auron, Philip E.**
**119 Wilson Drive**
**Framington, MA 01701(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.6/3.3.1)

CHEMICAL ABSTRACTS, vol. 96, no. 5, 1st February 1982, page 484, abstract no. 33132e, Columbus, Ohio, US; B.M. STADLER et al.: "Monoclonal antibodies against the interleukins", & PROG. CANCER RES. THER. 1981, 20(Lymphokines Thymic Horm.: Their Potential Util. Cancer Ther.), 69-76

CHEMICAL ABSTRACTS, vol. 99, no. 5, 1st August 1983, page 405, abstract no. 36951u, Columbus, Ohio, US; L.B. LACHMAN: "Human interleukin 1: purification and properties", & FED. PROC. FED. AM. SOC. EXP. BIOL. 1983, 42(9), 2639-45

PROCEEDINGS OF THE NATIONAL ACADEMY OF SCIENCE, vol. 81, no. 24, December 1984, pages 7907-7911, Washington, US; P.E. AURON et al.: "Nucleotide sequence of human monocyte interleukin 1 precursor cDNA"

BIOLOGICAL ABSTRACTS, RRM, abstract no. 28054236, Philadelphia, US; P.E. AURON et al.: "Molecular cloning of human interleukin-1 complementary DNA", & LYMPHOKINE RESEARCH (US) 1984, vol. 3, no. 4, p285

Fed. Proc.42 (1983) 2639-45

Proc. Natl. Acad. Sci. USA 84 (1987) 4572-4576

Proc. Natl. Acad. Sci. USA 83 (1986) 5243

J Immunol. 138 (1987) 1447-1450

J Immunol. 140 (1988) 2267-2273

Inventor: **Webb, Andrew C.**
6 Lovewell Road
Wellesley, MA 02181(US)
Inventor: **Gehrke, Lee**
11 Blueberry Circle
Framington, MA 01701(US)
Inventor: **Dinarello, Charles A.**
133 Mt. Vernon Street
Boston, MA 02108(US)
Inventor: **Rosenwasser, Lanny J.**
58 Sherburn Circle
Weston, MA 02193(US)
Inventor: **Rich, Alexander**
2 Walnut Avenue
Cambridge, MA 02140(US)
Inventor: **Wolff, Sheldon M.**
12 Lowell Road
Wellexley, MA 02181(US)

(74) Representative: **Perry, Robert Edward et al**
**GILL JENNINGS & EVERY**
Broadgate House
7 Eldon Street
London EC2M 7LH (GB)

## Description

It is well established that mononuclear phagocytes are required for antigen recognition and lymphocyte activation, and that they play a vital role in the immune response of the host to infectious, inflammatory and malignant disease. Several aspects of immunological function and host response to infection and injury are attributable to various proteins and other mediators released from stimulated mononuclear phagocytes (Dinarello, C.A. Rev. Inf. Dis. 6, 51-95 [1984]). These include leukocytic pyrogen (LP), a mediator of fever; leukocytic endogenous mediator (LEM), an inducer of several components of the acute phase response; lymphocyte activating factor (LAF), which augments both lymphocyte proliferation and lymphokine production; and mononuclear cell factor (MCF), which induces prostaglandin $E_2$ and collagenase synthesis in synovial cells. It has been demonstrated that LP and LAF activity co-purify and share common physical characteristics (Rosenwasser, L.J., Dinarello, C.A., and Rosenthal, A.S. J. Exp. Med. 150, 709-714 [1979]; Rosenwasser, L.J. and Dinarello, C.A. Cell. Immunol. 63, 134-142 [1981]; Murphy, P.A., Simon, P.L., and Willoughby, W.F. J. Immunol. 124, 2498-2501 [1980]). Similarly there is evidence that LP and LEM are closely related if not the same molecule (Kampschmidt, R.F. in The Physiologic and Metacolic Responses of the Host [eds M.C. Powanda and P.G. Canonico] 55-74 [Elsevier/North-Holland, Amsterdam, 1981]) and furthermore that LAF and MCF seem to be identical (Mizel, S.B., Dayer, J.M., Krane, S.M., and Mergenhagen, S.E. Proc. Natl. Acad. Sci. USA 78, 2474-2477 [1979]). The term interleukin-1 (IL-1) is now used to describe these varied biological activities, although it is presently unclear whether IL-1 represents a single substance or a family of related molecules. Prior to the subject invention the art had no knowledge of the nucleotide sequence coding for human IL-1. Though the art was aware of general cloning procedures, there is no teaching or suggestion in the prior art which could be used to ident and clone the nucleotide sequence coding for human IL-1.

## Brief Summary of the Invention

The subject invention concerns a nucleic acid comprising a nucleotide sequence coding for human IL-1, and fragments thereof, and to the polypeptides and peptides obtained. Specifically, the subject invention comprises the cloning of a cDNA synthesized by reverse transcription of poly(A)RNA isolated from adherent human monocytes stimulated with bacterial endotoxin. Injection of hybrid-selected poly(A)RNA into Xenopus laevis oocytes directed the synthesis of biologically active LAF. The nucleotide sequence, as well as immunoprecipitation of poly(A)RNA-directed reticulocyte translation, suggests that human IL-1 is initially synthesized as a precursor peptide with a molecular weight of 30,747. The subject invention also concerns truncated human IL-1 cDNA sequences which encode biologically-active human IL-1 proteins. These truncated cDNA sequences, and novel biologically-active human IL-1 proteins obtained therefrom, can be obtained by genetic engineering procedures using a clone containing the entire human IL-1 cDNA sequence as starting material. Specifically, with reference to Chart A, the nucleotide sequence located between residues 534 and 893 encode biologically-active IL-1 proteins. Within this range are two regions which encode biologically-active IL-1 proteins; i.e., (1) the nucleotide sequence located between residues 534 and 710, and (2) the nucleotide sequence located between residues 711 and 893.

## Detailed Description of the Invention

Monocytes were separated from lymphocytes in human peripheral blood mononuclear cells by using adherence to glass surfaces. The adherent monolayers (80-90% monocytes, as judged by microscopic examination of phagocytized staphylococcal particles) were stimulated with endotoxin. Total cellular nucleic acid was extracted from the adherent cell population, purified by centrifugation through CsCl (Chirgwin, J.M., Przybyla, A.E., MacDonald, R.J., and Rutter, W.J. Biochemistry 18, 5294-5299 [1979]), and enriched for poly(A)RNA by passage over oligo dT cellulose (Bantle, J.A., Maxwell, I.H., and Hahn, W.E. Analytical Biochem. 72, 413-427 [1976]).

Aliquots of poly(A)RNA were assayed for protein synthesis by in vitro translation using rabbit reticulocyte lysat containing $^{35}$S-methionine (Pelham, H.R.B. and Jackson, R.J. Eur. J. Biochem. 67, 242-256 [1976]). The translation products were immunoprecipitated using rabbit anti-human IL-1 antiserum (Dinarello, C.A., Renfer, L., and Wolff, S.M. J.Clin. Invest. 60, 465-472 [1977]; Dinarello, C.A., Renfer, L., and Wolff, S.M. Proc. Natl. Acad. Sci. USA 74, 4623-4627 [1977]) and staphylococcal protein A (Kessler S.W. J. Immunol. 115, 1617-1624 [1975]; Ivarie, R.D. and Jones, P.P. Analytical Biochem. 97, 24-35 [1979]). The immunoprecipitates were analyzed by SDS-polyacrylamide gel electrophoresis (SDS-PAGE) and autoradiography. The reticulocyte translation of stimulated monocyte-derived poly(A)RNA exhibits two intense immunoprecipitable bands, migrating with apparent molecular weights of 42,100 and 39,800, which are absent in the unstimulated poly(A)RNA preparation. A third, weaker band, migrating at 28,000 molecular weight also appears to be stimulation-specific. The measurement of the apparent molecular weights of these three proteins as determined by SDS-PAGE seems to be dependent upon the conditions of the electrophoresis. The proteins are represented as the following: 43 K band, 42,600 ± 1100; 35 K band, 34,678 ± 4800; 26 K band, 25,520 ± 3300.

Several poly(A)RNA preparations extracted from 12 h endotoxin-stimulated monocytes were pooled and fractionated by sucrose gradient sedimentation. Each fraction was precipated with ethanol, translated in a reticulocyte lysate, and analyzed by immunoprecipitation and electrophoresis as described above. RNA from selected fractions was also injected into oocytes. The culture medium from each batch of 20 oocytes was passed over Sephacryl S-200 and the eluted fractions were assayed for LAF activity as described above. It is clear that the majority of the activity clusters around the fractions containing the 35 K band (centering on fraction 13).

A cDNA library was prepared from endotoxin-stimulated monocyte poly(A)RNA using the technique and vector described by Okayama and Berg (Molec. Cell. Biol. 2, 161-170 [1982] This library was screened with 32p-labelled cDNA probes prepared from stimulated and unstimulated monocyte poly(A) as well as from RNA contained within fraction 12 of the sucrose gradient described above. As a result five cDNA clones representing three different size-classes were isolated on the basis that they were stimulation-specific and strongly related to material contained within fraction 12 of the sucrose gradient.

The cDNAs were used to produce hybrid-selected RNA (Maniatis, T., Fritsch, E.F., and Sambrook, J. Molecular Cloning: A Laboratory Manual, Cold Spring Harbor Laboratory, New York [1982]), which was analyzed by in vitro translation similar to that described above. cDNA from several clones hybridized to RNA which can be translated into a protein similar to that seen as a result of the translation of fraction 12 in the sucrose gradient profile.

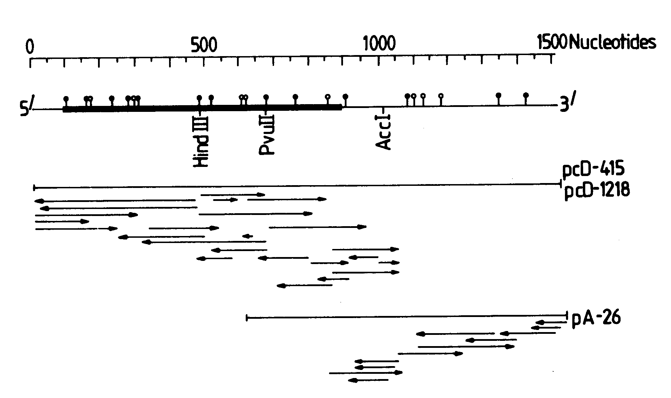

Clone pA-26 possessed the highest efficiency for hybrid-selection of the target RNA and similarly was associated with the strongest hybridization affinity for the cDNA probes that were used for screening. The cDNA transcript contained in pA-26 was sequenced as shown in the drawing and found to be approximately 920 base pairs in length. The single longest open reading frame for this sequence coded for a protein of ∿6,800 molecular weight. Since this did not correspond to the molecular size expected on the basis of the protein found in the reticulocyte translation, it was concluded that the cDNA transcript was not full length. Moreover, when nick-translated pA-26 plasmid DNA was hybridized to a Northern Blot (Rave, N. et al. Nuc. Acids Res. 2,815-825 [1979]; Maniatis, T., Fritsch, E.F., and Sambrook, J. Molecular Cloning: A Laboratory Manual, Cold Spring Harbor Laboratory, New York [1982]) of stimulated-monocyte poly(A)RNA, its complementary RNA appeared as a single band approximate 1600 nucleotides in length. Two additional cDNA libraries were constructed from 4-h and 12-h endotoxin-stimulated human monocyte poly(A)RNA using the newer Okayama and Berg procedure (Molec. Cell. Biol. 3, 280-289 [1983]). The result was that five 4-h and four 12-h clones hybridized to a DNA probe synthesized from clone pA-26. The cDNA inserts of these clones were of three different size classes. The largest insert (1560 b.p., as determined by agarose gel electrophoresis) was contained in both 12-h (one clone) and 4-h (four clones) libraries.

The 4-h clone pcD-415 hybridizes to an RNA preparation which has IL-1-like (LAF) biological activity when injected into Xenopus laevis oocytes. Furthermore this activity is absent from unstimulated monocyte poly(A)RNA and from hybrid-selected RNA made from the control cDNA 12-h clone pcD-1214, which is structurally unrelated to the pcD-415 clone. The elution position of this material on the Sephacryl S-200 column represents an approximate molecular weight of 20,000. This is in agreement with the molecular size of IL-1 isolated from stimulated monocyte media (Rosenwasser, L.J. and Dinarello, C.A. Cell. Immunol. 63, 134-142 [1981]).

From the above, we concluded that the three structurally-related clones pA-26, pcD-415, and pcD-1218 contain cDNA for human,monocyte IL-1. These clones were sequenced by the dideoxy chain termination technique following subcloning in various bacteriophage M13 cloning vectors. The drawing is a schematic summary of the strategy used for sequence determination. The top scale indicates the nucleotide positions relative to position 1 of the sequence as detailed in CHART A. The line immediately below the scale represents the extent of the sequence. The bold portion of the line delineates the predicted coding region for the IL-1 precursor. Restriction sites utilized in the sequencing procedure are indicated (open circles--Hae III and closed circles--Alu I). The arrows beneath each cDNA clone indicate the direction and extent of gel sequences read from the M13 subclones (Messing, J. and Vieira, J. Gene 19, 269-276 [1982]) (mp8 and mp9) using the dideoxy terminator method (Sanger, F., Nicklen, S., and Coulson, A.R. Proc. Natl. Acad. Sci. USA 74, 5463-5467 [1977]; Biggin, M.D., Gibson, T.J., and Hong, G.F. Proc. Natl. Acad. Sci. USA 80, 3963-3965 [1983]).

4

The consensus nucleotide sequence of human monocyte Il-1 cDNA and the predicted amino acid sequence of the protein is shown in CHART A. The apparent coding region corresponds to a molecular weight of 30,747 and is similar in size to the protein translated in reticulocyte lysates, described previously. Nucleotides are numbered with position 1 corresponding to the first nucleotide following the G tails resulting from cloning with the Okayama-Berg system. Boxed nucleotides represent a potential glycosylation site (Rubinstein, M.,Biochim. Biophys. Acta 695, 5-16 [1982]) and a potential polyadenylation signal (Proudfoot, N.J. and Brownlee, G.G. Nature 263, 211 [1976])

As disclosed above, our criteria for IL-1 identification are stringent, relying on data both from immunoprecipitation and biological assay of in vitro translation products. The polypeptide(s) in question must be stimulation specfic, immunoprecipitable, and demonstrate biological activity. Significantly, using these same criteria, little or no activity was observed in association with poly(A)RNA isolated from monocytes which were not stimulated by endotoxin. The reticulocyte lysate translation of poly(A)RNA extracted from stimulated cells reveals a major stimulation-specific polypeptide with a molecular weight which is similar to that predicted by the cDNA sequence. This corresponds to one of the two molecular weight species of IL-1 activity previously found in the medium of human monocytes following stimulation, as well as IL-1 recovered from human synovial fluid. In the subject disclosure, biological activity from micro-injected Xenopus oocytes and the activity found in stimulated monocyte media, co-elute from Sephacryl S-200 with an apparent molecular weight of 20,000, corresponding to the species reported by most investigators. This monocyte-derived protein can be isolated from endotoxin-stimulated monocytes in-cubated with $^{35}$S-methionine in culture yielding a biologically-active, radiolabelled molecule which migrates as a 22,000 molecular weight species when analyzed on the same SDS-PAGE system disclosed herein.

The cDNA nucleotide sequence suggests that the initial translation product is larger than the protein usually associated with IL-1 activity. We suggest, therefore, that a proteolytic "cascade" processes IL-1 following synthesis and/or release from stimulated monocytes. Throughout this proteolysis the molecule remains biologically active. Data derived from in vitro pulse-chase experiments support a precursor-product relationship between a large protein (approximately 31,000 molecular weight) and a series of smaller species which cross-react with our anti-serum. An arrow in CHART A located between $Ala_8$ and $Ser_9$ marks a potential signal sequence cleavage site somewhat similar to that predicted for Interleukin-2 (Taniguchi, T., Matsui, H., Fujita, T., Takaoa, C., Kashima, N., Yoshimoto, R., and Hamuro, J. Nature 302, 305-310 [1983]). A second arrow located between $Lys_{210}$ and $Met_{211}$ locates a potential cleavage site much like that described by Kronenberg et al. (Kronenberg, H.M., McDevitt, B.E., Majzoub, J.A., Sharp, P.A., Potts, J.T., and Rich, A. Proc. Natl. Acad. Sci. USA 76, 4981-4985 [1979]) for the cleavage of the pro-sequence from bovine proparathyroid hormone. These two potential cleavage sites delineate a putative peptide of 23,000 molecular weight, which is in reasonable agreement with the 15,000 to 20,000 size range reported by most investigators for the primary IL-1 activity.

Clone (plasmid) pcD-415, which contains the cDNA for human monocyte IL-1 was deposited in an E. coli HB101 host in the permanent collection of the Northern Regional Research Laboratory, U.S. Department of Agriculture, Peoria, Illinois, USA, on April 27, 1984. The culture was assigned the accession number NRRL B-15770 by the repository. This deposit is available to the public upon the grant of a patent disclosing it. The deposit is also available as required by foreign patent claws in countries wherein counterparts of the subject application, or its progeny, are filed. However, it should be understood that the availability of a deposit does not constitute a license to practice the subject invention in derogation of patent rights granted by governmental action.

Recombinant plasmid pcD-415 can be isolated from its E. coli HB101 host by well-known procedures, e.g., using cleared lysate-isopycnic density gradient procedures, and the like.

Also, it is within the skill of those in the art to vary the conditions disclosed herein for cloning the nucleotide sequence coding for human IL-1.

The cloned human IL-1 gene can be used to detect related DNAs in the human genome by well-known probe techniques. Further, unlimited amounts of nucleic acid comprising a nucleotide sequence coding human IL-1 can be made by the cloned human IL-1 gene of the subject invention. Still further, the IL-1 produced by the cloned gene of the subject invention can be used to induce the production of IL-2 by activating T-cells--IL-2 stimulates the T-cells to proliferate. As reported in Science, 221, 1362-1364, "Researchers from NIAID and the Food and Drug Administration (FDA), using a test tube assay, have recently found that interleukin-2 improved the function of T cells from six AIDS patients" (p. 1362).

In vitro, IL-1 activates neutrophils to degranulate and is also chemotactic. The most studied effects of IL-1 are on lymphocytes. IL-1 acts on B-cells, T-cells, as disclosed above, and NK-cells. On B-cells, IL-1 acts in conjunction with other B-cell activators as an adjuvant. It boosts B-cell proliferation and immunoglobulin synthesis (Lipsky, P.E., Thompson, P.A., Rosenwasser, L.J., Dinarello, C.A. J. Immunol. 130, 2708 [1983]; Falkoff, R.J.M., Muraguchi, A. Hong, J.X., Butler, J.L., Dinarello, .A., Fauci, A.S. J. Immunol. 131, 801 [1983]). On T-cells, IL-1 acts as a co-factor for T-cells to produce various lymphokines. IL-2 and leukocyte migration inhibitory factor have been studied as lymphokines which require a signal from IL-1 in the absence of monocytes or antigen presenting accessory cells (Mizel, S.B. Immunol. Rev. 63, 51 [1982]).

Another aspect of IL-1 is its inflammatory properties IL-1 has been isolated from the synovial fluid of patients with various forms of arthritis (Wood, D.D., Ihrie, E.J., Dinarello, C.A., Cohen, P.L. Arthr. Rheumat. 26, 975 [1983]), and its ability to increase collagenase and prostaglandin $E_2$ from synovial cells implicates IL-1 in the pathogenesis of several arthritides. In muscle tissue, IL-1 also induces prostaglandin $E_2$ but this leads to increased lysosomal protease activity and increases protein breakdown from muscle tissue (Baracos, V., Rodemann H.P., Dinarello, C.A., Goldberg, A.L. New Engl. J. Med. 308, 553 (1983]). In brain tissue, IL-1 also increases prostaglandin E and this plays a key role in the development of the febrile response (Dinarello, C.A. IN: Lymphokines, 4, ED). More recent research involves IL-1 in the induction of sleep (Kreuger, J.M., Walter, J., Dinarello, C.A., Wolff, S.M., Chedid, L. Am. J. Physiol. in press) and in fibroblast proliferation and collagen synthesis (Schmidt, J.A., Mizel, S.B., Cohen, D., Green, I. J. Immunol. 128, 2177 [1982]).

Because of its central role as a mediator of host immunological and defense functions, detection of IL-1 in different disease states is likely to shed light on certain pathological processes, and levels of IL-1 may indicate the severity of certain disease states where this is masked by special drugs. There is evidence that IL-1 production is reduced in human subjects with certain cancers (Hofmann, M.K., Pollack, S. IN: Interleukins, Lymphokines and Cytokines. ED Oppenheim, J.J., Cohen, S. Academic Press, 707-14 [1983]) and malnutrition (Keenan, R.A., Moldawer, L.L., Yang, R.D., Kawamura, I., Blackburn, G.L., Bistrian, B.R. J. Lab. Clin. Med. 100, 844 [1982]) and this has been supported by studies in animal models.

Use of IL-1 as an immunological reagent in humans or animals is likely because of its ability to stimulate T- and B-cells and increase immunoglobulin synthesis. In fact, IL-1 appears to be an excellent candidate for the body's endogenous adjuvant. Thus, it is possible to use IL-1 or parts of the IL-1 molecule in certain immunogens.

The subject invention, also, advantageously, provides novel biologically-active human IL-1 proteins through use of novel truncated human IL-1 cDNA sequences. As disclosed above the entire human IL-1 cDNA sequence is shown in CHART A. This sequence is the starting material for the preparation of the novel clones containing novel truncated IL-1 cDNA sequences.

Unlimited amounts of nucleic acid comprising nucleotide sequences coding for truncated human IL-1 can be made by the cloned human IL-1 cDNA of the subject invention. Further, the novel biologically-active human IL-1 proteins obtained via the cloned truncated human IL-1 cDNA sequences of the subject invention can be used in the same manner as native human IL-1, disclosed above.

The following examples are illustrative of the process and products of the subject invention but are not to be construed as limiting. All percentages are by weight and all solvent mixture proportions are by volume unless otherwise noted.

Example 1--Preparation of Poly(A)RNA

Human mononuclear cells (4-6 x $10^9$) were isolated from Ficoll-Hypaque gradient separation of plateletphoresis by-products. Cells were washed 4 x in 0.9% NaCl at 600 X g to remove platelets. Monocytes were plated into flat glass bottles in RPMI (Gibco) containing 1% (v/v) heat-inactivated human AB serum at a density of 1.25 x $10^6$ cells/$cm^2$ and allowed to adhere for 1.5 h at 37°. The bottles were then vigorously shaken and the non-adherent population drained and counted. The total number of adherent monocytes was determined by subtracting the non-adhering cells from the total cell count. Replacement (serum-free) RPMI contained 300 ng/ml E. coli endotoxin (Difco). After 12 h at 37°, the medium was drained and the adherent monolayer lysed by the addition of 6 M guanidinium thiocyanate (Chirgwin, J.M., Przybyla, A.E., MacDonald, R.J. and Rutter, W.J. Biochemistry 18, 5294-5299 [1979]). The lysate was frozen at -70° and thawed prior to layering onto CsCl cushions as previously described (See Chirgwin et al. supra). Poly(A)RNA was recovered from the crude nucleic acid pellets by binding to oligo(dT) cellulose twice (Bantle, J.A., Maxwell, I.H., and Hahn, W.E. Analytical Biochem. 72, 413-427 [1976]). Total RNA isolated from adherent monocytes ranged from 100-200 $\mu g/10^9$ cells of which poly(A)RNA consistently represented 5-7%. Preparation of poly(A)RNA from "unstimulated" mononuclear cells by the same procedure but without stimulation or adherence yielded about 500 $\mu g$ of total RNA/$10^9$ cells of which only 1-2% bound to oligo(dT) cellulose under the conditions used here.

Example 2--In Vitro Translation of Poly(A)RNA

Rabbit reticulocyte lysate was prepared, optimized and treated with micrococcal nuclease as described in Pelham, H.R.B. and Jackson, R.J. Eur. J. Biochem. 67, 242-256 (1976). Each translation contained 1 μg poly(A)RNA in the presence of 100 μCi $^{35}$S-methionine/ml. After incubation for 1 h at 37°, samples were immunoprecipitated according to the method of Kessler (J. Immunol. 115, 1617-1624 [1975]) with some modifications. During pre-clearing, 20 μl of normal rabbit serum (NRS) was added to each sample. This was followed by a 2 h incubation at 4°, after which 100 μl (i.e. 10% (w/v)) protein A (IgGsorb, The Enzyme Center, Boston, MA) was added. Samples were allowed to incubate an additional 1 h at room temperature and the IgGsorb was then pelleted by centrifugation in a clinical centrifuge for 10 min at maximum speed. Supernates were transferred to fresh tubes and incubated for 18 h at 4° with 20 μl of rabbit anti-human EP/LAF polyclonal serum (Dinarello, C.A., Renfer, L., and Wolff, S.M. J. Clin. Invest. 60, 465-472 [1977]). This antiserum was prepared by 20 monthly immunizations of 15 Kd human EP/LAF obtained after gel filtration (Dinarello, C.A., Goldin, N.P., and Wolff, S.M. J. Exp. Med. 139, 1369-1381 [1973]) and has anti-human EP/LAF but no anti-human IL-2 activity. Next, 100 μl IgGsorb was added to each tube, followed by incubation at room temperature for 1 h. IgGsorb was pelleted as described above and the pellet washed by vigorous vortexing with 1 ml aliquots (3X) 0.5% (v/v) Triton X-100. Antigens were then solubilized by the addition of 20 μl electrophoresis buffer containing 6% SDS (Laemli, U.K. Nature 227, 680-685 [1970]) and subsequent boiling for 5 min. Again IgGsorb was removed by microfuging for 3 min and the supernatant was then loaded onto a 17.5% polyacrylamide gel (15 x 17 x 0.15 cm) (see Laemli, supra) for electrophoresis at 35 mAmp for 5 h. Gels were treated with fluor (EnHance, KEN), dried and then exposed to photographic film (Kodak, XAR-5) for 24-72 h prior to development.

Example 3--Sucrose Gradient Fractionation of Stimulated Monocyte Poly(A)RNA

The sucrose gradient procedure is a modification of that described by Bleackley et al. (J. Immunol. 127, 2432-2435 [1981]). Poly(A)RNA (50 μg prepared from stimulated human monocytes) was dissolved in 475 μl of water, heated to 65° for 30 min, quench cooled on ice, and adjusted to : 50 mM Tris-HCl, pH 7.5; 0.1% lithium dodecyl sulfate; and 1 mM EDTA (TLE buffer). Samples were loaded onto isokinetic TLE-sucrose gradients (10-28%) and centrifuged in an SW41 rotor (Beckman) for 19 h (4°) at 35 Krpm. A parallel gradient was run with E. coli rRNA as markers. Gradients were fractionated (ISCO Model D) and ethanol precipitated. The RNA pellets were washed 2 X in 70% ethanol and then resuspended in 3 μl distilled water. Aliquots of each fraction were translated in rabbit reticulocyte lysate, immunoprecipitated and processed for electrophoresis and autoradiography as described above. In addition, selected fractions were micro-injected into Xenopus oocytes for assessment of biological activity also as described above. Stage V oocytes (Dumont, J.N. J. Morphol. 136, 153-180 [1972]) were manually defolliculated in Barth-X medium (Ford, C.C. and Gurdon, J.B. Embryol. Exp. Morph. 37, 203-209 [1977]) from the ovaries of adult Xenopus laevis (Nasco, WI) that had been stimulated with human chorionic gonadotropin (Sigma) 1-6 weeks beforehand. These oocytes were each micro-injected with 50 nl of poly(A)RNA solution in sterile distilled water (usually at 1-2 mg/ml). Controls were injected with a similar volume of distilled water only. Microinjected oocytes were incubated individually in round bottom microtiter wells containing 10 μl Barth-X to which was added antibiotics (per ml of culture media: 100 U penicillin; 100 μg streptomycin; 70 μg gentamicin; 2.5 μg amphotericin B) for 40-45 h at 20°. The Barth-X medium from batches of 20 oocytes was pooled and fractionated by gel filtration on a Sephacryl S-200 column (0.6 x 54 cm) equilibrated in RPMI containing 1% (v/v) heat-inactivated, fetal calf serum. Each fraction (about 1 ml) was placed in 3,500 m.w. cut-off dialysis tubing and concentrated five-fold by submerging in polyethylene glycol 8000 before assaying for LAF activity as reported in Rosenwasser and Dinarello, Cell. Immunol. 63, 134-142 (1981).

Example 4--Biological Activity of Hybrid-Selected RNA

Oocytes were processed as disclosed in Example 3, except that incubation was for 20 h and oocytes were incubated in microtiter plate wells at a density of 5 oocytes per well in 50 μl Barth-X media. LAF activity was assayed using a modification of the procedure described by Rosenwasser and Dinarello (supra), in which the mouse thymocyte cells were substituted with the D10 cell line described by Kaye, et al. (J. Exp. Med. 158, 836-856 [1983]).

Example 5--cDNA Clones

Three separate cDNA libraries were used to isolate the three cDNA clones shown in the drawing.The first, represented by clone pA-26, was constructed from 12h endotoxin-stimulated monocyte message using the original Okayama-Berg cloning vector system (Molec. Cell. Biol. 2, 161-170 [1982]). The second and third libraries, represented by clones pcD-415 and pcD-1218, are from, respectively, 4h and 12h endotoxin-stimulated monocyte message using the newer Okayama-Berg cloning vector system (Molec. Cell. Biol. 3, 280-289 [1983]). Libraries were each created using 2 $\mu$g of poly(A)RNA. A portion of the first library consisting of approximately 100 clones was screened with three different cDNA probes synthesized from stimulated and unstimulated monocyte poly(A)RNA as well as poly(A)RNA contained in fraction 12 of the sucrose gradient disclosed in Example 3. As a result five clones appeared to be more closely related to the enriched cDNA probe than to the unstimulated-RNA derived probe. The two clones containing the longest nucleotide sequence appeared to be identical on the basis of restriction mapping. One clone, pA-26, was subcloned in M13mp11 following treatment with Bal-31 exonuclease (Wei, C.F., Alianell, G.A., Bencen, G.H., and Gray, H.B. J. Biol. Chem. 258, 13506-13512 [1983]). The second and third cDNA libraries were screened with one of the M13 subclones of pA-26 cDNA using a hybridization probe primer (Hu, N. and Messing, J. Gene 17, 171 [1982]).

Example 6--Construction of a Plasmid Containing Truncated Human IL-1 cDNA That Codes for Proteins Corresponding to the DNA Sequences Located Between Nucleotide Positions 87 Through 677, and Positions 1355 Through 1396 Shown in Chart A.

The IL-1 cDNA sequence (Chart A) contains three unique restriction endonuclease digestion sites that can be used to construct plasmids containing specific deletions aimed at isolating essential domains of IL-1. Proceeding 5' to 3' in the directional sense of protein coding by the cDNA, these three sites are located respectively named and positioned as follows: Hind III (pos. 483); Pvu II (pos. 678); and Xmn I (pos. 1355) (Note: all restriction endonuclease sites presented here are referenced to the location of the first nucleotide on the 3' side of scission as read along the protein coding "sense" strand of the cDNA). In addition a unique Pst I restriction site located upstream from the cDNA sequence (pos. -16) can also be used.

The first plasmid construction deletes all IL-1 cDNA nucleotide sequence between the Pvu II and Xmn I sites, described above, and is as follows: Plasmid pL1, as described by H. Okayama and P. Berg (1983) Molec. Cell. Biol. 3:280-289, and which can be purchased from Pharmacia (Piscataway, NJ), is digested completely with Xmn I and Hind III restriction endonucleases. Three products which result can be resolved by agarose gel electrophoresis.

These products are approximately 518, 782, and 1544 base pairs in length. The 518 base pair fragment is isolated from the agarose gel using standard techniques. Another plasmid, e.g., pUC-8 (Messing, J. and Vieira, J. [1982] Gene 19:269-276), which can be purchased from Pharmacia, is used as a source of a DNA fragment which can be used as a linker segment to attach the Pst I restriction site located at one end of the 518 base pair fragment to a Hind III site which will be described below. pUC-8 contains a polycloning site with adjacent Pst I and Hind III sites and can be substituted for by other similar DNAs such as pUC-9 or M13mp8 or M13mp9 double stranded replicative forms. These DNAs can be purchased from Pharmacia. The pUC-8 plasmid is digested with Pst I and mixed with the 518 base pair fragment derived from pL1. The two fragments are ligated by T4 DNA ligase under conditions of excess pUC-8. Two products which result represent two different ligated orientations of the 518 fragment with respect to the linearized pUC-8. The two different orientations cannot easily be isolated from each other since each possesses the same molecular size (approximately 3660 base pairs). Isolation is accomplished by first digesting the 3660 base pair DNA mixture with Hind III endonuclease which causes the original mixture to be fragmented into 4 products of approximately 3650, 3140, 528, and 10 base pairs in length. These products can readily be resolved by standard agarose gel electrophoresis and the 528 base pair, pL1-derived, fragment (which now possess Hind III cohesive ends) is isolated.

The original human IL-1 cDNA plasmid (pcD-415), contained in the E. coli HB101 host, is isolated using standard plasmid preparation procedures. This plasmid is digested with both Pvu II and Xmn I restriction endonucleases to yield three products which are resolvable by agarose gel electrophoresis (approximate sizes are 675, 1633, and 2379 base pairs). The 1633 and 2379 base pair fragments are isolated from the gel and ligated in the presence of T4 DNA ligase to the pL1-derived, 528 base-pair fragment, described above. Two different plasmid constructs result, one of which has the proper orientation for the DNA fragments. The correct construct can readily be isolated by taking advantage of the fact that the ampicillin resistance gene contained within the pcD-415 plasmid will be properly reassembled only in the plasmid construction containing the desired IL-1 cDNA fragment orientation. Therefore E. coli HB101 cells transformed with the mixture containing both plasmids will only yield viable E. coli cells containing the proper construct when the cells are grown in the presence of ampicillin. From these cells the final construct (which is referred to as pcD-415ΔPvu/Xmn) can be isolated using standard plasmid isolation procedures. This plasmid contains truncated human IL-1 cDNA that codes for a protein corresponding to the DNA sequence located between nucleotide positions 87 through 677 and positions 1355 through 1396 shown in Chart A.

Example 7--Construction of a Plasmid Containing Truncated Human IL-1 cDNA that Codes for a Protein Corresponding to the DNA Sequence Located Between Nucleotide Positions 492 Through 893 Shown in Chart A.

This plasmid is constructed such that all the cDNA sequence between the upstream Pst I site and the Hind III site contained within the human IL-1 sequence is deleted. The starting material is plasmid pcD-415. Plasmid pcD-415 is digested with Hind III endonuclease and the two products (approximately 1016 and 3676 base pairs) resolved by agarose gel electrophoresis. The 3676 base pair fragment is isolated from the gel and mixed with the pL1-derived, 528 base pair (Hind III cohesive-ended) fragment prepared for use in constructing pcD-415ΔPvu/Xmn in Example 1. Ligation of these DNAs by T4 ligase results in two different plasmid products which can be purified and distinguished by transformation of E. coli HB101 cells and restriction mapping of the isolated plasmids. A Pvu II and Pst I double digestion permits clear identification of the product. The final product with the required deletion is referred to as pcD-415ΔPst/Hin. This plasmid contains a truncated human IL-1 cDNA that codes for a protein corresponding to the DNA sequence located between nucleotide positions 492 through 893 shown in Chart A.

Example 8--Construction of a Plasmid Containing Truncated Human IL-1 cDNA that Codes for Proteins Corresponding to the DNA Sequence Located Between Nucleotide Positions 492 Through 677 and Positions 1355 Through 1396 Shown in Chart A.

This construction is a combination of both deletions described above located within a single plasmid. The pcD-415ΔPst/Hin plasmid, described above, is digested with Pvu II and Xmn I to yield three agarose gel resolvable products (approximately 675, 1150, and 2379 base pairs). The 1150 and 2379 base pair fragments are isolated and ligated to yield two possible products which can be resolved in a fashion analogous to that described in Example 1 by selection of transformed E. coli HB101 in the presence of ampicillin. The final product with the required deletions is referred to as pcD-415ΔPst/Hin-ΔPvu/Xho. This plasmid contains a truncated human IL-1 cDNA that codes for proteins corresponding to the DNA sequence located between nucleotide positions 492 through 677 and positions 1355 through 1396 shown in Chart A.

The cDNA transcript can be obtained from the clones in essentially pure form by standard art methods. For example, the cDNA transcript in clone pcD-415 can be clipped from the plasmid by a BamHI-PstI double-digestion (Okayama, H. and Berg, P. Molec. Cell. Biol. 3, 280-289 [1983]) and isolated by standard procedures. The essentially pure cDNA thus obtained can be used for subcloning into a different transfer vector.

As is well known in the art, the amino acid sequence of a protein, e.g., IL-1, is determined by the nucleotide sequence of the DNA. Because of the redundancy of the genetic code, i.e., more than one coding nucleotide triplet (codon) can be used for most of the amino acids used to make proteins, different nucleotide sequences can code for a particular amino acid. Thus, the genetic code can be depicted as follows:

| | | | |
|---|---|---|---|
| Phenylalanine (Phe) | TTK | Histidine (His) | CAK |
| Leucine (Leu) | XTY | Glutamine (Gln) | CAJ |
| Isoleucine (Ile) | ATH | Asparagine (Asn) | AAK |
| Methionine (Met) | ATG | Lysine (Lys) | AAJ |
| Valine (Val) | GTL | Aspartic acid (Asp) | GAK |
| Serine (Ser) | QRS | Glutamic acid (Glu) | GAJ |
| Proline (Pro) | CCL | Cysteine (Cys) | TGK |
| Threonine (Thr) | ACL | Tryptophan (Try) | TGG |
| Alanine (Ala) | GCL | Arginine (Arg) | WGZ |
| Tyrosine (Tyr) | TAK | Glycine (Gly) | GGL |
| Termination signal | TAJ | | |
| Termination signal | TGA | | |

Key: Each 3-letter deoxynucleotide triplet corresponds to a trinucleotide of mRNA, having a 5'-end on the left and a 3'-end on the right. All DNA sequences given herein are those of the strand whose sequence corresponds to the mRNA sequence, with thymine substituted for uracil. The letters stand for the purine or pyrimidine bases forming the deoxynucleotide sequence.

A = adenine

G = guanine

C = cytosine

T = thymine

X = T or C if Y is A or G

X = C if Y is C or T

Y = A, G, C or T if X is C

Y = A or G if X is T

W = C or A if Z is A or G

W = C if Z is C or T

Z = A, G, C or T if W is C

Z = A or G if W is A

QR = TC if S is A, G, C or T

J = A or G

K = T or C

L = A, T, C or G

M = A, C or T

The above shows that the novel amino acid sequence of human IL-1 and human IL-proteins can be prepared by nucleotide sequences other than those disclosed herein. Functionally equivalent nucleotide sequences encoding the novel amino acid sequence of human IL-1, human IL-1 proteins, or fragments thereof having IL-1 activity, can be prepared by known synthetic procedures. Accordingly, the subject invention includes such functionally equivalent nucleotide sequences.

Thus the scope of the subject invention includes not only the specific nucleotide sequence depicted herein, but also all equivalent nucleotide sequences coding for molecules with substantially the same human IL-1 biological activity. The term "equivalent" is being used in its ordinary patent usage here as denoting a nucleotide sequence which performs substantially as the nucleotide sequence identified herein to produce molecules with substantially the same human IL-1 biological activity in essentially the same kind of hosts. Within this definition are subfragments which have human IL-1 biological activity.

It is well within the skill of those in the genetic engineering art to use the nucleotide squences encoding human IL-1 activity of the subject invention to produce human IL-1 via microbial processes. Fusing the sequences into an expression vector and transforming or transfecting into hosts, either eukaryotic (yeast or mammalian cells) or prokaryotic (bacterial cells), are standard procedures used in producing other well-known proteins, e.g., insulin, interferons, human growth hormone, and the like. Similar procedures, or obvious modifications thereof, can be employed to prepare human IL-1 proteins by microbial means or mammalian tissue-culture technology in accord with the subject invention.

As disclosed previously, the cDNA sequence in CHART A discloses, via two arrows, a cDNA sequence which itself codes for a peptide having human IL-1 activity. The isolation of this cDNA sequence is disclosed hereinafter. Upon isolation of the cDNA sequence in its essentially pure form it can be cloned by using the procedures described herein for the entire cDNA sequence coding for human IL-1. Those skilled in the art will appreciate the fact that the cDNA fragment depicted includes substantially biologically (human IL-1 activity) equivalent cDNA sequences, as defined above.

The process for isolating the cDNA fragment is as follows:

Plasmid pcD-415 is digested with Stu I and Xho I restriction endonucleases in order to generate a DNA fragment containing approximately 1370 bp of sequence. The sequence of interest (between positions 111-717, CHART A) is approximately centered within this fragment (approximately 350 nucleotides from each end). These excess terminal nucleotides can be removed using a time-controlled Bal 31 exonuclease limited digestion (Wei et al. J. Biol. Chem. 258, 13506-13512 [1983]). In this way a fragment containing the DNA sequence corresponding to that located between the two arrows in CHART A can be generated. Using a combination of techniques which are well known in the art, the resulting Bal 31 fragments can be subcloned and then selected using radioactive probes made from restriction endonuclease digestion fragments made from the original pcD-415 cDNA insert.

The nucleotide sequences obtained from IL-1 clone pcD-415 also can be prepared by a "gene machine" by procedures well known in the art. This is possible because of the disclosure of the nucleotide sequence. However, it is generally recognized in the art at this time that obtention of the desired nucleotide sequence from a clone, e.g., pcD-415, is the most expedient way to practice an invention such as disclosed and claimed herein.

The restriction enzymes disclosed can be purchased from Bethesda Research Laboratories, Gaithersburg, MD, or New England Biolabs, Beverly, MA. The enzymes are used according to the instructions provided by the supplier.

The various methods employed in the preparation of the plasmids and transformation of host organisms are well known in the art. These procedures are all described in Maniatis, T., Fritsch, E.F., and Sambrook, J. (1982) Molecular Cloning: A Laboratory Manual, Cold Spring Harbor Laboratory, New York. Thus, it is within the skill of those in the genetic engineering art to extract DNA from microbial cells, perform restriction enzyme digestions, electrophorese DNA fragments, tail and anneal plasmid and insert DNA, ligate DNA, transform cells, e.g., E. coli cells, prepare plasmid DNA, electrophorese proteins, and sequence DNA.

## CHART A

A C A A A C C T T T T C G A G G C A A A A G G C A A A A A A
10 20 30

G G C T G C T C T G G G A T T C T C T T C A G C C A A T C T
40 50 60

MET ALA
T C A A T G C T C A A G T G T C T G A A <u>G C A G C C A T G G</u>
70 80 90

GLU VAL PRO LYS LEU ALA ↓ SER GLU MET MET
C A G A A G T A C C T A A G C T C G C C A G T G A A A T G A
100 110 120

ALA TYR TYR SER GLY ASN GLU ASP ASP LEU
T G G C T T A T T A C A G T G G C A A T G A G G A T G A C T
130 140 150

PHE PHE GLU ALA ASP GLY PRO LYS GLN MET
T G T T C T T T G A A G C T G A T G G C C C T A A A C A G A
160 170 180

LYS CYS SER PHE GLN ASP LEU ASP LEU CYS
T G A A G T G C T C C T T C C A G G A C C T G G A C C T C T
190 200 210

PRO LEU ASP GLY GLY ILE GLN LEU ARG ILE
G C C C T C T G G A T G G C G G C A T C C A G C T A C G A A
220 230 240

SER ASP HIS HIS TYR SER LYS GLY PHE ARG
T C T C C G A C C A C C A C T A C A G C A A G G G C T T C A
250 260 270

12

## CHART A (page 2)

```
      GLN   ALA   ALA   SER   VAL   VAL   VAL   ALA   MET   ASP
    G G C A G G C C G C G T C A G T T G T T G T G G C C A T G G
                  280                 290                 300


      LYS   LEU   ARG   LYS   MET   LEU   VAL   PRO   CYS   PRO
    A C A A G C T G A G G A A G A T G C T G G T T C C C T G C C
                  310                 320                 330


      GLN   THR   PHE   GLN   GLU   ASN   ASP   LEU   SER   THR
    C A C A G A C C T T C C A G G A G A A T G A C C T G A G C A
                  340                 350                 360


      PHE   PHE   PRO   PHE   ILE   PHE   GLU   GLU   GLU   PRO
    C C T T C T T T C C C T T C A T C T T T G A A G A A G A A C
                  370                 380                 390


      ILE   PHE   PHE   ASP   THR   TRP   ASP   ASN   GLU   ALA
    C T A T C T T C T T C G A C A C A T G G G A T A A C G A G G
                  400                 410                 420


      TYR   VAL   HIS   ASP   ALA   PRO   VAL   ARG   SER   LEU
    C T T A T G T G C A C G A T G C A C C T G T A C G A T C A C
                  430                 440                 450


     ⌐ASN   CYS   THR ⌐  LEU   ARG   ASP   SER   GLN   GLN   LYS
    T G A A C T G C A C G C T C C G G G A C T C A C A G C A A A
                  460                 470                 480


      SER   LEU   VAL   MET   SER   GLY   PRO   TYR   GLU   LEU
    A A A G C T T G G T G A T G T C T G G T C C A T A T G A A C
                  490                 500                 510
```

CHART A (page 3)

```
      LYS     ALA     LEU     HIS     LEU     GLN     GLY     GLN     ASP     MET
T G A A A G C T C T C C A C C T C C A G G G A C A G G A T A
                  520                     530                     540


      GLU     GLN     GLN     VAL     VAL     PHE     SER     MET     SER     PHE
T G G A G C A A C A A G T G G T G T T C T C C A T G T C C T
                  550                     560                     570


      VAL     GLN     GLY     GLU     GLU     SER     ASN     ASP     LYS     ILE
T T G T A C A A G G A G A A G A A A G T A A T G A C A A A A
                  580                     590                     600


      PRO     VAL     ALA     LEU     GLY     LEU     LYS     GLU     LYS     ASN
T A C C T G T G G C C T T G G G C C T C A A G G A A A A G A
                  610                     620                     630


      LEU     TYR     LEU     SER     CYS     VAL     LEU     LYS     ASP     ASP
A T C T G T A C C T G T C C T G C G T G T T G A A A G A T G
                  640                     650                     660


      LYS     PRO     THR     LEU     GLN     LEU     GLU     SER     VAL     ASP
A T A A G C C C A C T C T A C A G C T G G A G A G T G T A G
                  670                     680                     690


      PRO     LYS     ASN     TYR     PRO     LYS     LYS     LYS ↓ MET     GLU
A T C C C A A A A A T T A C C C A A A G A A G A A G A T G G
                  700                     710                     720


      LYS     ARG     PHE     VAL     PHE     ASN     LYS     ILE     GLU     ILE
A A A A G C G A T T T G T C T T C A A C A A G A T A G A A A
                  730                     740                     750
```

## CHART A (page 4)

```
      ASN   ASN   LYS   LEU   GLU   PHE   GLU   SER   ALA   GLN
T C A A T A A C A A G C T G G A A T T T G A G T C T G C C C
            760                   770                   780


      PHE   PRO   ASN   TRP   TYR   ILE   SER   THR   SER   GLN
A G T T C C C C A A C T G G T A C A T C A G C A C C T C T C
            790                   800                   810


      ALA   GLU   ASN   MET   PRO   VAL   PHE   LEU   GLY   GLY
A A G C A G A A A C A T G C C C G T C T T C C T G G G A G
            820                   830                   840


      THR   LYS   GLY   GLY   GLN   ASP   ILE   THR   ASP   PHE
G G A C C A A A G G C G G C C A G G A T A T A A C T G A C T
            850                   860                   870


      THR   MET   GLN   PHE   VAL   SER   SER   ***
T C A C C A T G C A A T T T G T G T C T T C C T A A A G A G
            880                   890                   900


A G C T G T A C C C A G A G A G T C C T G T G C T G A A T G
            910                   920                   930


T G G A C T C A A T C C C T A G G G C T G G C A G A A A G G
            940                   950                   960


G A A C A G A A A G G T T T T T G A G T A C G G C T A T A G
            970                   980                   990


C C T G G A C T T T C C T G T T G T C T A C A C C A A T G C
            1000                  1010                  1020
```

15

CHART A (page 5)

C C A A C T G C C T G C C T T A G G G T A G T G C T A A G A
1030                  1040                  1050

G G A T C T C C T G T C C A T C A G C C A G G A C A G T C A
1060                  1070                  1080

G C T C T C T C C T T T C A G G G C C A A T C C C A G C C C
1090                  1100                  1110

T T T T G T T G A G C C A G G C C T C T C T C A C C T C T C
1120                  1130                  1140

C T A C T C A C T T A A A G C C C G C C T G A C A G A A A C
1150                  1160                  1170

C A G G C C A C A T T T T G G T T C T A A G A A A C C C T C
1180                  1190                  1200

C T C T G T C A T T C G C T C C C A C A T T C T G A T G A G
1210                  1220                  1230

C A A C C G C T T C C C T A T T T A T T T A T T T A T T T G
1240                  1250                  1260

T T T G T T T G T T T T G A T T C A T T G G T C T A A T T T
1270                  1280                  1290

A T T C A A A G G G G G C A A G A A G T A G C A G T G T C T
1300                  1310                  1320

**CHART A (page 6)**

```
G T A A A A G A G C C T A G T T T T T A A T A G C T A T G G
              1330              1340              1350

A A T C A A T T C A A T T T G G A C T G G T G T G C T C T C
              1360              1370              1380

T T T A A A T C A A G T C C T T T A A T T A A G A C T G A A
              1390              1400              1410

A A T A T A T A A G C T C A G A T T A T T T A A A T G G G A
              1420              1430              1440

A T A T T T A T A A A T G A G C A A A T A T C A T A C T G T
              1450              1460              1470

T C A A T G G T T C T C A A A T A A A C T T C A C T A A A A
              1480              1490              1500

AAAAAAA
```

**Claims**

1.  A recombinant DNA cloning vehicle which comprises cDNA including the gene for the IL-1 precursor having a molecular weight of 30747, or a functional equivalent thereof which, by virtue of the degeneracy of the genetic code, also codes for the said IL-1 precursor.

2.  A recombinant DNA cloning vehicle which comprises cDNA including a code for the amino-acid sequence:

EP 0 161 901 B1

```
Met Ala Glu Val Pro Lys Leu Ala Ser Glu Met Met Ala Tyr
Tyr Ser Gly Asn Glu Asp Asp Leu Phe Phe Glu Ala Asp Gly
Pro Lys Gln Met Lys Cys Ser Phe Gln Asp Leu Asp Leu Cys
Pro Leu Asp Gly Gly Ile Gln Leu Arg Ile Ser Asp His His
Tyr Ser Lys Gly Phe Arg Gln Ala Ala Ser Val Val Val Ala
Met Asp Lys Leu Arg Lys Met Leu Val Pro Cys Pro Gln Thr
Phe Gln Glu Asn Asp Leu Ser Thr Phe Phe Pro Phe Ile Phe
Glu Glu Glu Pro Ile Phe Phe Asp Thr Trp Asp Asn Glu Ala
Tyr Val His Asp Ala Pro Val Arg Ser Leu Asn Cys Thr Leu
Arg Asp Ser Gln Gln Lys Ser Leu Val Met Ser Gly Pro Tyr
Glu Leu Lys Ala Leu His Leu Gln Gly Gln Asp Met Glu Gln
Gln Val Val Phe Ser Met Ser Phe Val Gln Gly Glu Glu Ser
Asn Asp Lys Ile Pro Val Ala Leu Gly Leu Lys Glu Lys Asn
Leu Tyr Leu Ser Cys Val Leu Lys Asp Asp Lys Pro Thr Leu
Gln Leu Glu Ser Val Asp Pro Lys Asn Tyr Pro Lys Lys Lys
Met Glu Lys Arg Phe Val Phe Asn Lys Ile Glu Ile Asn Asn
Lys Leu Glu Phe Glu Ser Ala Gln Phe Pro Asn Trp Tyr Ile
Ser Thr Ser Gln Ala Glu Asn Met Pro Val Phe Leu Gly Gly
Thr Lys Gly Gly Gln Asp Ile Thr Asp Phe Thr Met Gln Phe
Val Ser Ser
```

3. A recombinant DNA cloning vehicle which comprises cDNA including a code for the amino-acid sequence:

```
Met Ala Glu Val Pro Lys Leu Ala Ser Glu Met Met Ala Tyr
Tyr Ser Gly Asn Glu Asp Asp Leu Phe Phe Glu Ala Asp Gly
Pro Lys Gln Met Lys Cys Ser Phe Gln Asp Leu Asp Leu Cys
Pro Leu Asp Gly Gly Ile Gln Leu Arg Ile Ser Asp His His
Tyr Ser Lys Gly Phe Arg Gln Ala Ala Ser Val Val Val Ala
Met Asp Lys Leu Arg Lys Met Leu Val Pro Cys Pro Gln Thr
Phe Gln Glu Asn Asp Leu Ser Thr Phe Phe Pro Phe Ile Phe
```

18

```
Glu Glu Glu Pro Ile Phe Phe Asp Thr Trp Asp Asn Glu Ala
Tyr Val His Asp Ala Pro Val Arg Ser Leu Asn Cys Thr Leu
Arg Asp Ser Gln Gln Lys Ser Leu Val Met Ser Gly Pro Tyr
Glu Leu Lys Ala Leu His Leu Gln Gly Gln Asp Met Glu Gln
Gln Val Val Phe Ser Met Ser Phe Val Gln Gly Glu Glu Ser
Asn Asp Lys Ile Pro Val Ala Leu Gly Leu Lys Glu Lys Asn
Leu Tyr Leu Ser Cys Val Leu Lys Asp Asp Lys Pro Thr Leu
Gln Asn Ser Ile Trp Thr Gly Val Leu Ser Leu Asn Gln Val
Leu
```

4. A recombinant DNA cloning vehicle which comprises cDNA including a code for the amino-acid sequence:

```
Met Ser Gly Pro Tyr Glu Leu Lys Ala Leu His Leu Gln Gly
Gln Asp Met Glu Gln Gln Val Val Phe Ser Met Ser Phe Val
Gln Gly Glu Glu Ser Asn Asp Lys Ile Pro Val Ala Leu Gly
Leu Lys Glu Lys Asn Leu Tyr Leu Ser Cys Val Leu Lys Asp
Asp Lys Pro Thr Leu Gln Leu Glu Ser Val Asp Pro Lys Asn
Tyr Pro Lys Lys Lys Met Glu Lys Arg Phe Val Phe Asn Lys
Ile Glu Ile Asn Asn Lys Leu Glu Phe Glu Ser Ala Gln Phe
Pro Asn Trp Tyr Ile Ser Thr Ser Gln Ala Glu Asn Met Pro
Val Phe Leu Gly Gly Thr Lys Gly Gly Gln Asp Ile Thr Asp
Phe Thr Met Gln Phe Val Ser Ser
```

5. A recombinant DNA cloning vehicle which comprises cDNA including a code for the amino-acid sequence:

```
Met Ser Gly Pro Tyr Glu Leu Lys Ala Leu His Leu Gln Gly
Gln Asp Met Glu Gln Gln Val Val Phe Ser Met Ser Phe Val
Gln Gly Glu Glu Ser Asn Asp Lys Ile Pro Val Ala Leu Gly
Leu Lys Glu Lys Asn Leu Tyr Leu Ser Cys Val Leu Lys Asp
Asp Lys Pro Thr Leu Gln Asn Ser Ile Trp Thr Gly Val Leu
Ser Leu Asn Gln Val Leu
```

6. Essentially pure cDNA having the nucleotide sequence shown in Chart A or a functional equivalent thereof which, by virtue of the degeneracy of the genetic code, also codes for the same amino-acid sequence.

7. Essentially pure cDNA having, in sequence, bases 1 to 677 and 1355 to 1507 shown in Chart A, or a functional equivalent thereof which, by virtue of the degeneracy of the genetic code, codes for the same amino-acid sequence.

8. Essentially pure cDNA having, in sequence, bases 482 to 1501 shown in Chart A, or a functional equivalent thereof which, by virtue of the degeneracy of the genetic code, codes for the same amino-acid sequence.

9. Essentially pure cDNA having, in sequence, bases 482 to 677 and 1355 to 1507 shown in Chart A, or a functional equivalent thereof which, by virtue of the degeneracy of the genetic code, codes for the same amino-acid sequence.

10. A recombinant DNA cloning vehicle which comprises cDNA as defined in any of claims 6 to 9.

11. A micro-organism transformed by a cloning vehicle according to any of claims 1, 2 and 10 (when appendant to claim 6).

12. A mammalian tissue culture cell line transfected by a cloning vehicle according to any of claims 1, 2 and 10 (when appendant to claim 6).

13. Recombinant plasmid pcD-415 as available in E. coli HB101 (pcD-415) deposited with NRRL under the accession number B-15770.

14. A bacterium transformed by a recombinant plasmid according to claim 13.

15. E. coli HB101 (pcD-415) deposited with NRRL under the accession number B-15770.

16. A process for preparing human IL-1b, i.e. the IL-1 precursor having a molecular weight of 30747, or a polypeptide having the amino-acid sequence defined in any of claims 2 to 5, which comprises culturing a microbe hosting a cloning vehicle according to any of claims 1 to 5 and 13.

17. A polypeptide having the amino-acid sequence defined in any of claims 2 to 5.

18. An antibody to human IL-1$\beta$, i.e. the IL-1 precursor having a molecular weight of 30747, which is as produced in response to an essentially pure immunogenic peptide, or a conjugate comprising a peptide, having the amino-acid sequence defined in claim 2, or a fragment thereof.

**Patentansprüche**

1. Rekombinantes DNA-Klonierungsvehikel, welches cDNA umfaßt, die das Gen für den IL-1-Vorläufer mit einem Molekulargewicht von 30747 einschließt, oder ein funktionelles Äquivalent davon, welches aufgrund der Degeneration des genetischen Codes auch für den IL-1-Vorläufer codiert.

2. Rekombinantes DNA-Klonierungsvehikel, welches cDNA umfaßt, die einen Code für die folgende Aminosäuresequenz aufweist:

```
Met Ala Glu Val Pro Lys Leu Ala Ser Glu Met Met Ala Tyr
Tyr Ser Gly Asn Glu Asp Asp Leu Phe Phe Glu Ala Asp Gly
Pro Lys Gln Met Lys Cys Ser Phe Gln Asp Leu Asp Leu Cys
Pro Leu Asp Gly Gly Ile Gln Leu Arg Ile Ser Asp His His
Tyr Ser Lys Gly Phe Arg Gln Ala Ala Ser Val Val Val Ala
Met Asp Lys Leu Arg Lys Met Leu Val Pro Cys Pro Gln Thr
Phe Gln Glu Asn Asp Leu Ser Thr Phe Phe Pro Phe Ile Phe
Glu Glu Glu Pro Ile Phe Phe Asp Thr Trp Asp Asn Glu Ala
Tyr Val His Asp Ala Pro Val Arg Ser Leu Asn Cys Thr Leu
Arg Asp Ser Gln Gln Lys Ser Leu Val Met Ser Gly Pro Tyr
Glu Leu Lys Ala Leu His Leu Gln Gly Gln Asp Met Glu Gln
Gln Val Val Phe Ser Met Ser Phe Val Gln Gly Glu Glu Ser
Asn Asp Lys Ile Pro Val Ala Leu Gly Leu Lys Glu Lys Asn
Leu Tyr Leu Ser Cys Val Leu Lys Asp Asp Lys Pro Thr Leu
Gln Leu Glu Ser Val Asp Pro Lys Asn Tyr Pro Lys Lys Lys
Met Glu Lys Arg Phe Val Phe Asn Lys Ile Glu Ile Asn Asn
Lys Leu Glu Phe Glu Ser Ala Gln Phe Pro Asn Trp Tyr Ile
Ser Thr Ser Gln Ala Glu Asn Met Pro Val Phe Leu Gly Gly
Thr Lys Gly Gly Gln Asp Ile Thr Asp Phe Thr Met Gln Phe
Val Ser Ser.
```

**3.** Rekombinantes DNA-Klonierungsvehikel, welches cDNA umfaßt, die einen Code für die folgende Aminosäuresequenz aufweist:

```
Met Ala Glu Val Pro Lys Leu Ala Ser Glu Met Met Ala Tyr
Tyr Ser Gly Asn Glu Asp Asp Leu Phe Phe Glu Ala Asp Gly
Pro Lys Gln Met Lys Cys Ser Phe Gln Asp Leu Asp Leu Cys
Pro Leu Asp Gly Gly Ile Gln Leu Arg Ile Ser Asp His His
Tyr Ser Lys Gly Phe Arg Gln Ala Ala Ser Val Val Val Ala
Met Asp Lys Leu Arg Lys Met Leu Val Pro Cys Pro Gln Thr
Phe Gln Glu Asn Asp Leu Ser Thr Phe Phe Pro Phe Ile Phe
Glu Glu Glu Pro Ile Phe Phe Asp Thr Trp Asp Asn Glu Ala
Tyr Val His Asp Ala Pro Val Arg Ser Leu Asn Cys Thr Leu
Arg Asp Ser Gln Gln Lys Ser Leu Val Met Ser Gly Pro Tyr
Glu Leu Lys Ala Leu His Leu Gln Gly Gln Asp Met Glu Gln
Gln Val Val Phe Ser Met Ser Phe Val Gln Gly Glu Glu Ser
Asn Asp Lys Ile Pro Val Ala Leu Gly Leu Lys Glu Lys Asn
Leu Tyr Leu Ser Cys Val Leu Lys Asp Asp Lys Pro Thr Leu
Gln Asn Ser Ile Trp Thr Gly Val Leu Ser Leu Asn Gln Val
Leu,
```

4. Rekombinantes DNA-Klonierungsvehikel, welches cDNA umfaßt, die einen Code für die folgende Aminosäuresequenz aufweist:

```
Met Ser Gly Pro Tyr Glu Leu Lys Ala Leu His Leu Gln Gly
Gln Asp Met Glu Gln Gln Val Val Phe Ser Met Ser Phe Val
Gln Gly Glu Glu Ser Asn Asp Lys Ile Pro Val Ala Leu Gly
Leu Lys Glu Lys Asn Leu Tyr Leu Ser Cys Val Leu Lys Asp
Asp Lys Pro Thr Leu Gln Leu Glu Ser Val Asp Pro Lys Asn
Tyr Pro Lys Lys Lys Met Glu Lys Arg Phe Val Phe Asn Lys
Ile Glu Ile Asn Asn Lys Leu Glu Phe Glu Ser Ala Gln Phe
Pro Asn Trp Tyr Ile Ser Thr Ser Gln Ala Glu Asn Met Pro
Val Phe Leu Gly Gly Thr Lys Gly Gly Gln Asp Ile Thr Asp
Phe Thr Met Gln Phe Val Ser Ser.
```

5. Rekombinantes DNA-Klonierungsvehikel, welches cDNA umfaßt, die einen Code für die folgende Aminosäuresequenz aufweist:

```
Met Ser Gly Pro Tyr Glu Leu Lys Ala Leu His Leu Gln Gly
Gln Asp Met Glu Gln Gln Val Val Phe Ser Met Ser Phe Val
Gln Gly Glu Glu Ser Asn Asp Lys Ile Pro Val Ala Leu Gly
Leu Lys Glu Lys Asn Leu Tyr Leu Ser Cys Val Leu Lys Asp
Asp Lys Pro Thr Leu Gln Asn Ser Ile Trp Thr Gly Val Leu
Ser Leu Asn Gln Val Leu.
```

EP 0 161 901 B1

**6.** Im wesentlichen reine cDNA mit der in Karte A gezeigten Nucleotidsequenz oder ein funktionelles Äquivalent davon, welches aufgrund der Degeneration des genetischen Codes ebenfalls für dieselbe Aminosäuresequenz codiert.

**7.** Im wesentlichen reine cDNA, welche aufeinanderfolgend die in Karte A gezeigten Basen 1 bis 677 und 1355 bis 1507 aufweist, oder ein funktionelles Äquivalent davon, welches aufgrund der Degeneration des genetischen Codes für dieselbe Aminosäuresequenz codiert.

**8.** Im wesentlichen reine cDNA, welche aufeinanderfolgend die in Karte A gezeigten Basen 482 bis 1501 aufweist, oder ein funktionelles Äquivalent davon, welches aufgrund der Degeneration des genetischen Codes für dieselbe Aminosäuresequenz codiert.

**9.** Im wesentlichen reine cDNA, welche aufeinanderfolgend die in Karte A gezeigten Basen 482 bis 677 und 1355 bis 1507 aufweist, oder ein funktionelles Äquivalent davon, welches aufgrund der Degeneration des genetischen Codes für dieselbe Aminosäuresequenz codiert.

**10.** Rekombinantes DNA-Klonierungsvehikel, welches die in irgendeinem der Ansprüche 6 bis 9 definierte cDNA umfaßt.

**11.** Mikroorganismus, transformiert mit einem Klonierungsvehikel nach irgendeinem der Ansprüche 1, 2 und 10 (wenn zurückbezogen auf Anspruch 6).

**12.** Säugetiergewebekultur-Zellinie, transfiziert mit einem Klonierungsvehikel nach irgendeinem der Ansprüche 1, 2 und 10 (wenn zurückbezogen auf Anspruch 6).

**13.** Rekombinantes Plasmid pcD-415, verfügbar in E. coli HB101 (pcD-415), hinterlegt bei NRRL unter der Hinterlegungsnummer B-15770.

**14.** Bakterium, transformiert mit einem rekombinanten Plasmid nach Anspruch 13.

**15.** E. coli HB101 (pcD-415), hinterlegt bei NRRL unter der Hinterlegungsnummer B-15770.

**16.** Verfahren zur Herstellung von Human-IL-1$\beta$, d.h. des IL-1-Vorläufers mit einem Molekulargewicht von 30747, oder eines Polypeptids mit einer in irgendeinem der Ansprüche 2 bis 5 definierten Aminosäuresequenz, welches die Kultivierung einer Mikrobe umfaßt, die ein Klonierungsvehikel nach irgendeinem der Ansprüche 1 bis 5 und 13 beherbergt.

**17.** Polypeptid mit der in irgendeinem der Ansprüche 2 bis 5 definierten Aminosäuresequenz.

**18.** Antikörper gegen Human-IL-1$\beta$, d.h. den IL-1-Vorläufer mit einem Molekulargewicht von 30747, welcher als Antwort auf ein im wesentlichen reines immunogenes Peptid oder ein Konjugat, umfassend ein Peptid, das die in Anspruch 2 definierte Aminosäuresequenz aufweist, oder ein Fragment davon produziert wurde.

**Revendications**

**1.** Véhicule de clonage d'ADN recombiné qui comprend un ADNc contenant le gène codant pour le précurseur d'IL-1 ayant une masse moléculaire de 30747, ou un de ses équivalents fonctionnels qui, en vertu de la dégénérescence du code génétique, code aussi pour ledit précurseur d'IL-1.

**2.** Véhicule de clonage d'ADN recombine qui contient un ADNc comprenant un code pour la séquence d'aminoacides:

23

```
Met Ala Glu Val Pro Lys Leu Ala Ser Glu Met Met Ala Tyr
Tyr Ser Gly Asn Glu Asp Asp Leu Phe Phe Glu Ala Asp Gly
Pro Lys Gln Met Lys Cys Ser Phe Gln Asp Leu Asp Leu Cys
Pro Leu Asp Gly Gly Ile Gln Leu Arg Ile Ser Asp His His
Tyr Ser Lys Gly Phe Arg Gln Ala Ala Ser Val Val Val Ala
Met Asp Lys Leu Arg Lys Met Leu Val Pro Cys Pro Gln Thr
Phe Gln Glu Asn Asp Leu Ser Thr Phe Phe Pro Phe Ile Phe
Glu Glu Glu Pro Ile Phe Phe Asp Thr Trp Asp Asn Glu Ala
Tyr Val His Asp Ala Pro Val Arg Ser Leu Asn Cys Thr Leu
Arg Asp Ser Gln Gln Lys Ser Leu Val Met Ser Gly Pro Tyr
Glu Leu Lys Ala Leu His Leu Gln Gly Gln Asp Met Glu Gln
Gln Val Val Phe Ser Met Ser Phe Val Gln Gly Glu Glu Ser
Asn Asp Lys Ile Pro Val Ala Leu Gly Leu Lys Glu Lys Asn
Leu Tyr Leu Ser Cys Val Leu Lys Asp Asp Lys Pro Thr Leu
Gln Leu Glu Ser Val Asp Pro Lys Asn Tyr Pro Lys Lys Lys
Met Glu Lys Arg Phe Val Phe Asn Lys Ile Glu Ile Asn Asn
Lys Leu Glu Phe Glu Ser Ala Gln Phe Pro Asn Trp Tyr Ile
Ser Thr Ser Gln Ala Glu Asn Met Pro Val Phe Leu Gly Gly
Thr Lys Gly Gly Gln Asp Ile Thr Asp Phe Thr Met Gln Phe
Val Ser Ser
```

3. Véhicule de clonage d'ADN recombiné qui contient un ADNc comprenant un code pour la séquence d'aminoacides:

```
Met Ala Glu Val Pro Lys Leu Ala Ser Glu Met Met Ala Tyr
Tyr Ser Gly Asn Glu Asp Asp Leu Phe Phe Glu Ala Asp Gly
Pro Lys Gln Met Lys Cys Ser Phe Gln Asp Leu Asp Leu Cys
Pro Leu Asp Gly Gly Ile Gln Leu Arg Ile Ser Asp His His
Tyr Ser Lys Gly Phe Arg Gln Ala Ala Ser Val Val Val Ala
Met Asp Lys Leu Arg Lys Met Leu Val Pro Cys Pro Gln Thr
```

```
Phe Gln Glu Asn Asp Leu Ser Thr Phe Phe Pro Phe Ile Phe
Glu Glu Glu Pro Ile Phe Phe Asp Thr Trp Asp Asn Glu Ala
Tyr Val His Asp Ala Pro Val Arg Ser Leu Asn Cys Thr Leu
Arg Asp Ser Gln Gln Lys Ser Leu Val Met Ser Gly Pro Tyr
Glu Leu Lys Ala Leu His Leu Gln Gly Gln Asp Met Glu Gln
Gln Val Val Phe Ser Met Ser Phe Val Gln Gly Glu Glu Ser
Asn Asp Lys Ile Pro Val Ala Leu Gly Leu Lys Glu Lys Asn
Leu Tyr Leu Ser Cys Val Leu Lys Asp Asp Lys Pro Thr Leu
Gln Asn Ser Ile Trp Thr Gly Val Leu Ser Leu Asn Gln Val
Leu
```

**4.** Véhicule de clonage d'ADN recombiné qui contient un ADNc comprenant un code pour la séquence d'aminoacides:

```
Met Ser Gly Pro Tyr Glu Leu Lys Ala Leu His Leu Gln Gly
Gln Asp Met Glu Gln Gln Val Val Phe Ser Met Ser Phe Val
Gln Gly Glu Glu Ser Asn Asp Lys Ile Pro Val Ala Leu Gly
Leu Lys Glu Lys Asn Leu Tyr Leu Ser Cys Val Leu Lys Asp
Asp Lys Pro Thr Leu Gln Leu Glu Ser Val Asp Pro Lys Asn
Tyr Pro Lys Lys Lys Met Glu Lys Arg Phe Val Phe Asn Lys
Ile Glu Ile Asn Asn Lys Leu Glu Phe Glu Ser Ala Gln Phe
Pro Asn Trp Tyr Ile Ser Thr Ser Gln Ala Glu Asn Met Pro
Val Phe Leu Gly Gly Thr Lys Gly Gly Gln Asp Ile Thr Asp
Phe Thr Met Gln Phe Val Ser Ser
```

**5.** Véhicule de clonage d'ADN recombiné qui contient un ADNc comprenant un code pour la séquence d'aminoacides:

```
Met Ser Gly Pro Tyr Glu Leu Lys Ala Leu His Leu Gln Gly
Gln Asp Met Glu Gln Gln Val Val Phe Ser Met Ser Phe Val
Gln Gly Glu Glu Ser Asn Asp Lys Ile Pro Val Ala Leu Gly
Leu Lys Glu Lys Asn Leu Tyr Leu Ser Cys Val Leu Lys Asp
Asp Lys Pro Thr Leu Gln Asn Ser Ile Trp Thr Gly Val Leu
Ser Leu Asn Gln Val Leu
```

**6.** ADNc essentiellement pur ayant la séquence de nucléotides présentée dans le diagramme A ou un de ses équivalents fonctionnels qui, en vertu de la dégénérescence du code génétique, code aussi pour la même séquence d'aminoacides.

**7.** ADNc essentiellement pur ayant, en séquence, les bases 1 à 677 et 1355 à 1507 indiquées dans le diagramme A, ou un de ses équivalents fonctionnels qui, en vertu de la dégénérescence du code génétique, code pour la même séquence d'aminoacides.

**8.** ADNc essentiellement pur ayant, en séquence, les bases 482 à 1501 indiquées dans le diagramme A, ou un de ses équivalents fonctionnels qui, en vertu de la dégénérescence du code génétique, code pour la même séquence d'aminoacides.

**9.** ADNc essentiellement pur ayant, en séquence, les bases 482 à 677 et 1355 à 1507 indiquées dans le diagramme A, ou un de ses équivalents fonctionnels qui, en vertu de la dégénérescence du code génétique, code pour la même séquence d'aminoacides.

**10.** Véhicule de clonage d'ADN recombiné qui contient un ADNc tel que défini dans l'une quelconque des revendications 6 à 9.

**11.** Microorganisme transformé par un véhicule de clonage selon l'une quelconque des revendications 1, 2 et 10 (lorsqu'elle dépend de la revendication 6).

**12.** Lignée cellulaire de culture de tissu de mammifère transfectée par un véhicule de clonage selon l'une quelconque des revendications 1, 2 et 10 (lorsqu'elle dépend de la revendication 6).

**13.** Plasmide recombiné pcD-415 tel que disponible dans E. coli HB101 (pcD-415) déposé auprès du NRRL sous le numéro d'accès B-15770.

**14.** Bactérie transformée par un plasmide recombiné selon la revendication 13.

**15.** E. coli HB101 (pcD-415) déposée auprès du NRRL sous le numéro d'accès B-15770.

**16.** Procédé de préparation d'IL-1b humaine, c'est-à-dire du précurseur d'IL-1 ayant une masse moléculaire de 30747, ou d'un polypeptide ayant la séquence d'aminoacides définie dans l'une quelconque des revendications 2 à 5, qui comprend la culture d'un microbe hôte d'un véhicule de clonage selon l'une quelconque des revendications 1 à 5 et 13.

**17.** Polypeptide ayant la séquence d'aminoacides définie dans l'une quelconque des revendications 2 à 5.

**18.** Anticorps dirigé contre l'IL-1$\beta$ humaine, c'est-à-dire le précurseur d'IL-1 ayant une masse moléculaire de 30747, tel qu'il est produit en réponse à un peptide immunogène essentiellement pur, ou à un conjugué comprenant un peptide, ayant la séquence d'aminoacides définie dans la revendication 2, ou un de ses fragments.